# EUROPEAN PATENT APPLICATION

(11) **EP 3 939 559 A1**
(43) Date of publication of application: **19.01.2022**
(21) Application number: 21185244.7
(22) Date of filing: 13.07.2021
(51) Int. Cl.: A61K 6/17, A61K 6/60, A61K 6/889, A61K 6/898, C04B 28/28

(54) **DENTAL GLASS IONOMER CEMENT COMPOSITIONS CONTAINING POLYSACCHARIDE NANOFIBER**

(30) Priority: 16.07.2020 JP 2020122119
(71) Applicant: Kabushiki Kaisha Shofu, Kyoto-shi Kyoto 605-0983 (JP)
(72) Inventor: SHIMOSOYAMA, Shun, Kyoto, 605-0983 (JP); SAKAMOTO, Shuji, Kyoto, 605-0983 (JP); KIMOTO, Katsuya, Kyoto, 605-0983 (JP)
(74) Representative: Eisenführ Speiser

(57) **Abstract**

An object of the present invention is to provide a dental glass ionomer cement composition having high safety to a human body and sufficient working time, in which mechanical properties, mixing ability in hand mixing and dischargeability out of a container after mechanical kneading are simultaneously improved with satisfactory balance compared with the prior art. Disclosed is a dental glass ionomer cement composition including 0.001 to 3% by weight of (a) a polysaccharide nanofiber having an aspect ratio of 100 or more and an average fiber diameter of 100 nm or less.

## Description

### BACKGROUND OF THE INVENTION

### Field of the Invention

The present invention relates to dental glass ionomer cement compositions such as a glass ionomer cement for dental filling and a glass ionomer cement for dental luting.

### Description of the Related Art

In a dental practice, in order to restore aesthetically and functionally a tooth in which a form was partially lost by caries or breakages, a direct restoration in which a glass ionomer cement for dental filling is filled into a tooth and an indirect restoration in which a dental prosthesis device is bonded or adhered to a tooth using a glass ionomer cement for dental luting has been performed.

A dental glass ionomer cement generally includes an acid reactive glass powder, polyalkenoic acid and water as main components. When an acidic compound such as polyalkenoic acid acts on an acid reactive glass powder in the presence of water, polyvalent metal ions (Al³⁺, Ca²⁺, Sr²⁺, etc.) are eluted from the acid reactive glass powder, and the polyalkenoic acid is ionically cross-linked with the polyvalent metal ions, resulting in setting. In a dental glass ionomer cement composition consisting of such constituents, there have been known, as a general method for improving the mechanical properties, a method for increasing the mixing ratio of the acid reactive glass powder, a method for increasing the specific surface area of the acid reactive glass powder, a method for increasing the mixing ratio of the polyalkenoic acid, a method for increasing the molecular weight of the polyalkenoic acid and a method for decreasing the mixing ratio of water.

There has hitherto been proposed a technique of mixing various fiber materials for the purpose of improving the mechanical properties of a dental material.

For example, JP 2020-29423 A proposes a composition for dental material containing microfibrous cellulose and a glass ionomer cement, which has excellent mechanical strength while improving the ability to elute a fluoride ion.

However, JP 2020-29423 A does not propose at all a problem that mechanical strength is improved while exhibiting satisfactory kneadability and dischargeability out of a container after kneading.

JP 2000-256038 A proposes a technique of mixing a glass fiber as an inorganic fiber with a glass powder for dental glass ionomer cement.

However, when an inorganic fiber is mixed with a dental glass ionomer cement composition, there is a concern about adverse effects on a human body, such as carcinogenicity, and therefore the user and the manufacturer had to pay careful attention to handling thereof.

The dental glass ionomer cement composition has a problem that mechanical properties such as compressive strength are not enough. However, when the mechanical properties of the dental glass ionomer cement composition are attempted to be improved using a general method such as a method for increasing the mixing ratio of the acid reactive glass powder, any method leads to an increased viscosity of a kneaded material, resulting in deterioration of mixing ability in hand mixing and dischargeability out of a container after mechanical kneading. In other words, it was difficult to simultaneously improve mechanical properties, kneadability and dischargeability out of a container with satisfactory balance.

### SUMMARY OF THE INVENTION

Therefore, an object of the present invention is to provide a dental glass ionomer cement composition having high safety to a human body and sufficient working time, in which mechanical properties, mixing ability in hand mixing and dischargeability out of a container after mechanical kneading are simultaneously improved with satisfactory balance compared with the prior art.

As a result of intensive studies in light of the above problems, the present inventors have found that mechanical properties are improved without compromising safety to a human body and while maintaining sufficient working time by mixing a polysaccharide nanofiber with a dental glass ionomer cement composition. The present inventors have also found that there is obtained a dental glass ionomer cement composition having a decreased viscosity of a kneaded material and also excellent mixing ability in hand mixing and dischargeability out of a container after mechanical kneading, and thus the present invention has been completed.

The present invention is directed to a dental glass ionomer cement composition including 0.001 to 3% by weight of (a) a polysaccharide nanofiber having a fiber aspect ratio of 100 or more and an average fiber diameter of 100 nm or less.

The polysaccharide nanofiber (a) may be at least one selected from the group consisting of a cellulose nanofiber, a chitin nanofiber and a chitosan nanofiber.

The viscosity (B-type viscometer, HBDV-I Prime manufactured by Brookfield Engineering Laboratories Inc., 60 rpm) of an aqueous 2% by weight solution of the polysaccharide nanofiber (a) at 25°C may be within a range of 500 to 10,000.

The dental glass ionomer cement composition may include:
0.001 to 3% by weight of (a) a polysaccharide nanofiber,
60 to 80% by weight of (b) an acid reactive glass powder,
5 to 20% by weight of (c) a polyalkenoic acid, and
10 to 25% by weight of (d) water,
based on the total weight of the composition.

According to the present invention, it is possible to provide a dental glass ionomer cement composition having high safety to a human body, sufficient working time and further significantly improved mechanical properties. Since the dental glass ionomer cement composition of the present invention has a low viscosity of a kneaded material, the dental glass ionomer cement composition exhibits not only excellent mixing ability in hand mixing but also satisfactory dischargeability out of a container after mechanical kneading, and thus high mechanical properties can be exhibited stably.

### DETAILED DESCRIPTION OF THE INVENTION

Each component in the dental glass ionomer cement composition of the present invention will be described in detail.

The polysaccharide nanofiber (a) which can be used in the dental glass ionomer cement composition of the present invention may use, as raw materials, cornstarch, potato starch, tapioca starch, dextrin, starch, glycogen, cellulose, chitin, chitosan, agarose, carrageenan, heparin, hyaluronic acid, pectin, xyloglucan, glucomannan, tamarind seed gum, guar gum, locust bean gum, arabic gum, karaya gum, konjac mannan, carrageenan, agar, alginic acid, xanthan gum, gellan gum, *Agrobacterium succinoglycan,* carboxymethyl cellulose and the like. The nanofiber in the present invention refers to a fibrous substance having a fiber aspect ratio of 100 or more.

It is possible to use, as the polysaccharide nanofiber (a), a nanofiber using at least one selected from the group consisting of cellulose, chitin and chitosan as a raw material among the raw materials, namely, at least one selected from the group consisting of a cellulose nanofiber, a chitin nanofiber and a chitosan nanofiber, from the viewpoint that a nanofiber having high strength can be obtained.

The cellulose nanofiber is a general term of a fibrous substance obtained by defibering an aggregate of microfibrils (cellulose microfibrils) having a diameter 3 to 4 nm formed from a bundle of tens of cellulose molecules, which are main components of a plant cell wall, by chemical treatment or mechanical treatment into a diameter of 100 nm or less. The cellulose nanofiber is an organic substance, and thus has a small specific gravity, and has a structure in which cellulose molecules in the fiber are highly crystallized; therefore, the cellulose nanofiber has a high elastic modulus equivalent to that of an aramid fiber. It is also known that since the coefficient of thermal expansion is small, thermal stability is also high, and biocompatibility is also excellent.

The chitin nanofiber is a nanofiber consisting of a chitin molecule, which is a main component of the outer shell of crustaceans, such as crabs and shrimps, or insects, or the cell wall of fungi including mushrooms. Like the cellulose nanofiber, the chitin nanofiber is characterized by being lightweight and having a high elastic modulus. The chitin is known to cure wounds and burns, and is a material which is particularly expected in the aspect of biocompatibility, as can be seen from the fact that medical materials utilizing its effect have been commercialized. The chitosan is obtained by deacetylating chitin by alkali treatment, and the chitosan nanofiber also has similar characteristics to that of the chitin nanofiber.

A defibering method to obtain the polysaccharide nanofiber (a) is not particularly limited, and it is possible to use a polysaccharide nanofiber defibered by any method including mechanical defibering methods such as a high pressure homogenizer method, a microfluidizer method, a high shear mixing method, a ball mill grinding method and a water jet production method, and chemical defibering methods such as an alkali treatment method, an acid treatment method and a TEMPO oxidation treatment method without any limitation. It is possible to use, as the polysaccharide nanofiber (a), a polysaccharide nanofiber which is functionalized to impart desired properties, but there is no any limitation on whether the polysaccharide nanofiber is functionalized or not. The polysaccharide nanofiber (a) thus obtained can be used alone or in combination of several types thereof.

Regarding the polysaccharide nanofiber (a), the viscosity of an aqueous 2% by weight solution at 25°C may be within a range of 500 to 10,000 mPa·s. The viscosity as used herein means a viscosity calculated from a value measured with a B-type viscometer (B-type viscometer, HBDV-I Prime manufactured by Brookfield Engineering Laboratories Inc., 60 rpm). When the viscosity is outside the above range, it might be difficult to obtain an effect to improve kneadability, dischargeability out of a container and mechanical properties.

The polysaccharide nanofiber (a) may have a specific surface area within a range of 50 to 300 m²/g. The specific surface area as used herein means a specific surface area calculated by measuring a dried nanofiber powder by a BET specific surface area measurement method. When the specific surface area is outside the above range, it might be difficult to obtain an effect to improve the kneadability, dischargeability out of a container and mechanical properties.

The polysaccharide nanofiber (a) may have a degree of polymerization within a range of 100 to 1,000. The degree of polymerization as used herein means a degree of polymerization calculated from a value measured by a viscosity method using a copper ethylenediamine solution. When the degree of polymerization is outside the above range, it might be difficult to obtain an effect to improve kneadability, dischargeability out of a container and mechanical properties.

The polysaccharide nanofiber (a) may have an average fiber diameter within a range of 1 to 100 nm. The average fiber diameter as used herein means an average fiber diameter calculated by image analysis of an observation image obtained with a scanning electron microscope. When the average fiber diameter is outside the above range, it might be difficult to obtain an effect to improve the kneadability, dischargeability out of a container and mechanical properties.

Regarding the content of the polysaccharide nanofiber (a), the polysaccharide nanofiber is mixed within a range of 0.001 to 3% by weight based on the whole dental glass ionomer cement composition. The content is preferably within a range of 0.001 to 0.5% by weight, and may be within a range of 0.003 to 0.5% by weight. When the content of the polysaccharide nanofiber (a) is outside the range of 0.001 to 3% by weight, it might be impossible to obtain an effect to improve the kneadability, dischargeability out of a container and mechanical properties.

The acid reactive glass powder (b) which can be used in the dental glass ionomer cement composition of the present invention includes an acid reactive element such as a metallic element, and a fluorine element. Since the acid reactive glass powder (b) includes an acid reactive element, the acid-base reaction of the acid reactive glass powder with the acid group contained in the polyalkenoic acid (c) progresses in the presence of water. Specific examples of the acid reactive element include, but are not limited to, sodium, potassium, calcium, strontium, barium, lanthanum, aluminum and zinc. One or more types of these acid reactive elements may be contained, and the content thereof is not particularly limited.

Furthermore, the acid reactive glass powder (b) may include an X-ray impermeable element in order to impart an X-ray contrast property to the dental glass ionomer cement composition of the present invention. Specific examples of the X-ray impermeable element include, but are not limited thereto, strontium, lanthanum, zirconium, titanium, yttrium, ytterbium, tantalum, tin, tellurium, tungsten and bismuth. Other elements contained in the acid reactive glass powder (b) are not particularly limited, and the acid reactive glass powder (b) in the present invention may include various elements.

Examples of the acid reactive glass powder (b) include, but are not limited to, aluminosilicate glass, borosilicate glass, aluminoborate glass, boro aluminosilicate glass, phosphate glass, borate glass and silica glass in which the above-mentioned acid reactive element, fluorine element and X-ray impermeable element are contained.

Furthermore, a particle shape of the acid reactive glass powder (b) is not particularly limited, and any particle shape such as spherical, needle-like, plate-like, ground-like and scaly shapes may be used without any limitation. These acid reactive glass powders (b) may be used alone or in combination of several types thereof.

A method for producing these acid reactive glass powders (b) is not particularly limited, and those produced by any production method such as a melting method, a vapor phase method and a sol-gel method may be used without any problem. Of these, an acid reactive glass powder (b) produced by a melting method or a sol-gel method, which can easily control a type of an element and the content thereof, is preferably used.

The acid reactive glass powder (b) may be ground to use in order to obtain a desired particle diameter. A grinding method is not particularly limited, and an acid reactive glass powder obtained by grinding which use any of wet or dry grinding methods may be used. Specifically, the acid reactive glass powder (b) may be ground by a high speed rotating mill such as a hammer mill and a turbo-mill, a container driving medium mill such as a ball mill and a vibration mill, a medium stirring mill such as a sand grinder and attritor, a jet mill, a bead mill and the like, and it is possible to appropriately adjust the average particle diameter according to the application or purpose of usage of the dental glass ionomer cement composition of the present invention.

For example, when the dental glass ionomer cement composition of the present invention is used as a material for filling or abutment building, since high mechanical properties are required, the 50% particle diameter of the acid reactive glass powder (b) may be within a range of 0.01 to 30 µm, and preferably 0.01 to 10 µm.

When the dental glass ionomer cement composition of the present invention is used as a material for luting, since a thin film thickness is required, the 50% particle diameter of the acid reactive glass powder (b) may be within a range of 0.01 to 10 µm, and preferably 0.01 to 5 µm.

When the 50% particle diameter of the acid reactive glass powder (b) is less than 0.01 µm, the surface area of the acid reactive glass powder (b) increases and it becomes impossible to contain the acid reactive glass powder (b) in a large amount into the composition, and therefore there is a risk that deterioration of mechanical properties may be caused. There is a case that the working time may be shortened or the viscosity of the kneaded material may increase to cause deterioration of kneadability, and therefore no uniform kneaded material can be obtained and the designed properties cannot be exhibited. There is also a case that the dischargeability out of a container may deteriorate.

In the case of using as a material for filling, when the 50% particle diameter of the acid reactive glass powder (b) exceeds 30 µm, the surface of the material after polishing becomes rough, coloring or discoloration may be caused. Furthermore, in the case of using as a material for luting, when the 50% particle diameter of the acid reactive glass powder (b) exceeds 10 µm, since the film thickness becomes thick, a prosthesis device to be adhered is lifted and therefore there is a risk that the intended fit of the prosthesis device cannot be obtained.

The acid reactive glass powder (b) may be treated with various treatments such as a surface treatment, a heat treatment, a aggregating treatment in a liquid phase or a vapor phase, microcapsulation in which a particle is enclosed with an organic substance, or grafting in which a surface is functionalized with an organic substance to such a range that the acid-base reaction of the acid reactive glass powder (b) with the polyalkenoic acid (c) is not adversely affected, in order to adjust operability, setting properties, mechanical properties and the like of the dental glass ionomer cement composition of the present invention. These treatments may be performed alone or in combination of several types, without any problem. Of these, a surface treatment or a heat treatment is suitable because it is easy to control various properties and it is superior in productivity.

Specific examples of the surface treatment method of the acid reactive glass powder (b) include washing with an acid such as phosphoric acid or acetic acid, surface treatment with an acidic compound such as tartaric acid or polycarboxylic acid, a surface treatment with a fluoride such as aluminum fluoride and a surface treatment with a silane compound such as γ-mercaptopropyl trimethoxy silane or tetramethoxy silane. The surface treatment method which can be used in the present invention is not limited to the above-mentioned methods, and these surface treatment methods may be used alone or in combination of a plurality thereof.

Specific examples of the heat treatment method of the acid reactive glass powder (b) include a treatment method in which heating is performed using an electric furnace for 1 hour to 72 hours within a range of 100°C to 800°C. The heat treatment method which can be used in the present invention is not limited to the above-mentioned method, and a single treatment or multi-stage treatment can be used with respect to the treatment process.

The content of the acid reactive glass powder (b) is preferably 60 to 80% by weight based on the whole dental glass ionomer cement composition. When the content of the acid reactive glass powder (b) is less than 60% by weight, mechanical properties may deteriorate. When the content of the acid reactive glass powder (b) exceeds 80% by weight, there is a case that the working time may be shortened or the viscosity of the kneaded material may increase to cause deterioration of kneadability, and therefore no uniform kneaded material can be obtained and the designed properties cannot be exhibited. There is also a case that the dischargeability out of a container may deteriorate.

The polyalkenoic acid (c) which can be used in the dental glass ionomer cement composition of the present invention may be used without any limitation as long as it is a homopolymer or a copolymer of alkenoic acid having at least one carboxy group in a molecule, such as unsaturated monocarboxylic acid, unsaturated dicarboxylic acid and unsaturated tricarboxylic acid. Furthermore, the polyalkenoic acid (c) may be a copolymer of a polymerizable monomer having no acid group in a molecule and alkenoic acid, without any problem.

Specific examples of the alkenoic acid which can be used to obtain the polyalkenoic acid (c) include, but are not limited to, (meth)acrylic acid, 2-chloroacrylic acid, 3-chloro(meth)acrylic acid, 2-cyanoacrylic acid, aconitic acid, mesaconic acid, maleic acid, maleic anhydride, itaconic acid, itaconic anhydride, fumaric acid, glutaconic acid, citraconic acid, utraconic acid, 1-butene-1,2,4-tricarboxylic acid and 3-butene-1,2,3-tricarboxylic acid. Of these, the polyalkenoic acid (c) which is synthesized from only acrylic acid as a starting raw material, or the polyalkenoic acid (c) which is synthesized from two or more types as a starting raw material, such as acrylic acid and maleic acid, acrylic acid and maleic anhydride, acrylic acid and itaconic acid, and acrylic acid and 3-butene-1,2,3-tricarboxylic acid, may be used.

A polymerization method to obtain various polyalkenoic acids is not particularly limited, and a polymer polymerized by any method such as solution polymerization, suspension polymerization and emulsion polymerization may be used without any limitation. A polymerization initiator and a chain transfer agent used at the time of synthesis of a polymer may be appropriately selected in order to obtain a desired polymer. The polyalkenoic acid (c) thus obtained may be used alone or in combination of several types thereof.

The polyalkenoic acid (c) may have a weight average molecular weight within a range of 30,000 to 300,000. The weight average molecular weight as used herein means an average molecular weight calculated based on the molecular weight distribution measured by gel permeation chromatography. When the weight average molecular weight of the polyalkenoic acid (c) is less than 30,000, mechanical properties may deteriorate. When the weight average molecular weight of the polyalkenoic acid (c) exceeds 300,000, there is a case that the working time may be shortened or the viscosity of the kneaded material may increase to cause deterioration of kneadability, and therefore no uniform kneaded material can be obtained and the designed properties cannot be exhibited. There is also a case that the dischargeability out of a container may deteriorate.

The content of the polyalkenoic acid (c) may be 5 to 20% by weight based on the whole dental glass ionomer cement composition. When the content of the polyalkenoic acid (c) is less 5% by weight, mechanical properties may deteriorate. When the content of the polyalkenoic acid (c) exceeds 20% by weight, there is a case that the working time may be shortened or the viscosity of the kneaded material may increase to cause deterioration of kneadability, and therefore no uniform kneaded material can be obtained and the designed properties cannot be exhibited. There is also a case that the dischargeability out of a container may deteriorate.

The water (d) which can be used in the dental glass ionomer cement composition of the present invention functions as a solvent to dissolve the polyalkenoic acid (c) and is a component for dispersing a metal ion eluted from the acid reactive glass powder (b) to induce cross-linking reaction with the polyalkenoic acid (c).

Any water may be used as the water (d) as long as it does not contain impurities which adversely affect the setting ability and mechanical properties of the dental glass ionomer cement composition without any limitation. Specifically, distilled water or ion-exchanged water may be used.

The content of the water (d) may be 10 to 25% by weight based on the whole dental glass ionomer cement composition. When the content of the water (d) is less than 10% by weight, there is a case that the working time may be shortened or the viscosity of the kneaded material may increase to cause deterioration of kneadability, and therefore no uniform kneaded material can be obtained and the designed properties cannot be exhibited. There is also a case that the dischargeability out of a container may deteriorate. When the content of the water (d) exceeds 25% by weight, mechanical properties may deteriorate.

An acidic compound can be contained in the dental glass ionomer cement composition of the present invention, for the purpose of adjusting the working time and the setting time. Specific examples of these acidic compounds include carboxylic acid compounds such as tartaric acid, citric acid, maleic acid, fumaric acid, malic acid, aconitic acid, tricarballylic acid, itaconic acid, 1-butene-1,2,4-tricarboxylic acid and 3-butene-1,2,3-tricarboxylic acid, phosphoric acid compounds such as phosphoric acid, pyrophosphoric acid and tripolyphosphoric acid, and metal salts of these acidic compounds. These acid compounds may be used alone or in combination of several types thereof. The content of the acid compound may be within a range of 0.1 to 15% by weight based on the whole dental glass ionomer cement composition.

A surfactant can be contained in the dental glass ionomer cement composition of the present invention to such a range that various properties are not influenced, for the purpose of improving kneadability. The surfactant which can be used in the dental glass ionomer cement composition of the present invention may be any of an ionic surfactant and a nonionic surfactant.

Specific examples of the anionic surfactant in the ionic surfactant include aliphatic carboxylic acid metal salts such as sodium stearate, sulfated aliphatic carboxylic acid metal salts such as sodium dioctyl sulfosuccinate, and metal salts of higher alcohol sulfate ester such as sodium stearyl sulfate. Examples of the cationic surfactant include an adduct of higher alkylamine and ethylene oxide, amines made from lower amine, and alkyltrimethylammonium salts such as lauryltrimethylammonium chloride. Furthermore, examples of the amphoteric surfactant include metal salts of higher alkylaminopropionic acid such as sodium stearylaminopropionate, and betaines such as lauryldimethylbetaine.

Examples of the nonionic surfactant include polyethylene glycol type or polypropylene glycol type in which ethylene oxide or propylene oxide is added to higher alcohols, alkyl phenols, fatty acids, higher aliphatic amines or aliphatic amides, or polyhydric alcohol type in which polyhydric alcohols, diethanolamines or saccharides are ester-bonded to a fatty acid.

The aforementioned surfactants are not limited thereto, and may be used without any limitation. These surfactants may be used alone or in combination of several types thereof.

The content of the surfactant in the dental glass ionomer cement composition of the present invention may be within a range of 0.001 to 5% by weight based on the whole composition.

Furthermore, when the dental glass ionomer cement composition of the present invention includes a paste form, a thickener can be contained to such a range that various properties are not influenced, for the purpose of adjusting the paste properties.

The thickener used in the dental glass ionomer cement composition of the present invention may be any of an inorganic thickener and an organic thickener. Examples of the inorganic thickener include fumed silica, calcium carbonate, calcium silicate, magnesium silicate, and a clay mineral such as saponite, montmorillonite, beidellite, vermiculite, sauconite, stevensite, hectorite, smectite, nectite and sepiolite.

Examples of the organic thickener include methyl cellulose, hydroxyethyl cellulose, hydroxymethyl cellulose, hydroxypropyl cellulose, carboxymethyl cellulose, sodium carboxymethyl cellulose, calcium carboxymethyl cellulose, carboxypolymethylene, sodium alginate, propylene glycol alginate ester, sodium polyacrylate, starch, starch sodium glycolate, starch phosphate ester, polyvinylpyrrolidone, carboxyvinyl polymer, khaya gum, arabic gum, karaya gum, guar gum and the like. These thickeners may be used alone or as a mixture of two or more types thereof. However, as the above-mentioned organic thickener, a fibrous thickener cannot be used. The fiber as used herein means an aggregate of microfibrils formed from a bundle of tens of molecules, having an aspect ratio of 100 or more.

The content of the thickener in the paste contained in the dental glass ionomer cement composition of the present invention may be within a range of 0.001 to 10% by weight.

Furthermore, a non-acid reactive powder can be contained in the dental glass ionomer cement composition of the present invention to such a range that various properties are not adversely affected, for the purpose of adjusting operability, mechanical properties or setting properties.

As the non-acid reactive powder used in the dental glass ionomer cement composition of the present invention, any non-acid reactive powder may be used without limitation as long as the non-acid reactive powder does not contain an element which forms a chelate bond with an acid group of the polyalkenoic acid (c). Examples of the non-acid reactive powder include known dental fillers such as an inorganic filler, an organic filler and an organic-inorganic complex filler, and these may be used alone or in combination of several types thereof. Of these, it is particularly preferable that an inorganic filler is used. Furthermore, a shape of these non-acid reactive powders is not particularly limited, and any particle shape such as spherical, needle-like, plate-like, ground-like and scaly shapes and an aggregate thereof may be used, but are not limited thereto. An average particle diameter of these non-acid reactive powders is not particularly limited, and may be within a range of 0.001 to 30 µm.

Specific examples of the inorganic filler include, but are not limited to, quartz, amorphous silica, ultrafine silica, various glasses not containing an element which forms a chelate bond with an acid group or an alkali metal salt of an acid group (including glass by a melting method, synthetic glass by a sol-gel method, glass produced by a vapor phase reaction and the like), silicon nitride, silicon carbide, boron carbide and the like.

The content of the non-acid reactive powder contained in the dental glass ionomer cement composition of the present invention may be within a range of 0.001 to 20% by weight based on the whole composition.

The dental glass ionomer cement composition of the present invention is provided in various forms such as powder material/liquid material, paste/liquid material and paste/paste as long as the acid reactive glass powder (b) and the polyalkenoic acid (c) do not coexist in the presence of the water (d). A ratio of these combinations is not particularly limited, and is preferably within a range of 0.5/1.0 to 8.0/1.0 by weight, more preferably 1.0/1.0 to 6.0/1.0 by weight, and still more preferably 2.0/1.0 to 4.0/1.0 by weight.

As the form of the powder material/liquid material,
a combination of a powder material containing the acid reactive glass powder (b) and a liquid material containing the polysaccharide nanofiber (a), the polyalkenoic acid (c) and the water (d),
a combination of a powder material containing the polysaccharide nanofiber (a) and the acid reactive glass powder (b) and a liquid material containing the polyalkenoic acid (c) and the water (d),
a combination of a powder material containing the polysaccharide nanofiber (a) and the acid reactive glass powder (b) and a liquid material containing the polysaccharide nanofiber (a), the polyalkenoic acid (c) and the water (d),
a combination of a powder material containing the acid reactive glass powder (b) and the polyalkenoic acid (c) and a liquid material containing the polysaccharide nanofiber (a) and the water (d),
a combination of a powder material containing the acid reactive glass powder (b) and the polyalkenoic acid (c) and a liquid material containing the polysaccharide nanofiber (a), the polyalkenoic acid (c) and the water (d),
a combination of a powder material containing the polysaccharide nanofiber (a), the acid reactive glass powder (b) and the polyalkenoic acid (c) and a liquid material containing the water (d),
a combination of a powder material containing the polysaccharide nanofiber (a), the acid reactive glass powder (b) and the polyalkenoic acid (c) and a liquid material containing the polysaccharide nanofiber (a), the polyalkenoic acid (c) and the water (d),
   and the like are provided, but is not limited thereto.

As the form of the paste/liquid material,
a combination of a paste containing the acid reactive glass powder (b) and the water (d) and a liquid material containing the polysaccharide nanofiber (a), the polyalkenoic acid (c) and the water (d),
a combination of a paste containing the polysaccharide nanofiber (a), the acid reactive glass powder (b) and the water (d) and a liquid material of the polyalkenoic acid (c) and the water (d),
a combination of a paste containing the polysaccharide nanofiber (a), the acid reactive glass powder (b) and the water (d) and a liquid material containing the polysaccharide nanofiber (a), the polyalkenoic acid (c) and the water (d), and the like are provided, but is not limited thereto.

As the form of the paste/paste,
a combination of a first paste containing the acid reactive glass powder (b) and the water (d) and a second paste containing the polysaccharide nanofiber (a), the polyalkenoic acid (c) and the water (d),
a combination of a first paste of the polysaccharide nanofiber (a), the acid reactive glass powder (b) and the water (d) and a second paste containing the polyalkenoic acid (c) and the water (d),
a combination of a first paste containing the polysaccharide nanofiber (a), the acid reactive glass powder (b) and the water (d) and a second paste containing the polysaccharide nanofiber (a), the polyalkenoic acid (c) and the water (d),
   and the like are provided, but is not limited thereto.

Furthermore, the dental glass ionomer cement composition of the present invention may optionally contain components such as preservatives, antimicrobial materials, coloring pigments and other conventionally known additives, if necessary.

### Examples

The present invention will be specifically described by way of Examples, but the present invention is not limited to these Examples. Test methods which evaluated the performance of the dental glass ionomer cement compositions prepared in Examples and Comparative Examples are as follows.

### [Compressive Strength]

A compressive strength was measured by the following procedures in accordance with ISO 9917-1:2007. In the environment of 23°C and a humidity of 50%, the dental glass ionomer cement composition of the present invention was kneaded in the proportions mentioned in Examples, and then the kneaded material thus obtained was filled in a stainless steel die (4ϕ × 6 mm: cylindrical) to allow to stand for 1 hour in a thermohygrostat at 37°C and a humidity of 100%. After 1 hour, the test specimen was taken out from the die, and immersed in ion-exchanged water at 37°C. After 24 hours from the end of the kneading, the test specimen was taken out, and an Instron universal tester (model: 5567A) was used to measure the compressive strength at a crosshead speed of 1 mm/min. In the present specification, when the compressive strength is 200 MPa or more, the composition was judged to have a high compressive strength.

### [Working Time]

In the environment of 23°C and a humidity of 50%, the dental glass ionomer cement composition of the present invention was kneaded in the proportions mentioned in Examples, and then the fluidity of the kneaded material thus obtained was confirmed using a spatula. Duration from the time of start of kneading as the base point to the time when the fluidity of the kneaded material is lost and filling and imparting a form become impossible was defined as the working time. In the present specification, when the working time is 1 minute and 30 seconds or more, the composition was judged to have sufficient working time.

### [Kneadability]

In the environment of 23°C and a humidity of 50%, the dental glass ionomer cement composition of the present invention was kneaded in the proportions mentioned in Examples, and at this time, the kneadability was evaluated using the following rating criteria: A: It could be easily kneaded; B: Although the viscosity of the kneaded material was slightly high, it could be kneaded without a problem; C: The viscosity of the kneaded material was high, and it was difficult to knead. In the present specification, when the kneadability was evaluated as A or B, the composition was judged to exhibit satisfactory kneadability.

### [Dischargeability Out Of Container]

In the environment of 23°C and a humidity of 50%, the dental glass ionomer cement composition of the present invention was filled in a dedicated container in the proportions mentioned in Examples, and mechanical kneading was performed using an automatic kneading device (Ultramat 2, manufactured by SDI Limited). After kneading, a piston attached to the back end of the container was pushed to discharge the kneaded material from the tip of the nozzle attached to the front end of the container, and at this time, the dischargeability out of a container was evaluated using the following rating criteria: A: It could be easily discharged; B: Although the viscosity of the kneaded material was slightly high, it could be discharged without a problem; C: The viscosity of the kneaded material was high, and it was difficult to discharge. In the present specification, when the dischargeability out of a container was evaluated as A or B, the composition was judged to exhibit satisfactory dischargeability out of a container.

When, as a result of evaluation of the above-mentioned four types of performance, a high compressive strength, sufficient working time, satisfactory kneadability and satisfactory dischargeability out of a container are exhibited, the composition was judged to have desired properties as a dental glass ionomer cement.

The components used for preparing the dental glass ionomer cement compositions of Examples and Comparative Examples, and their abbreviations are shown below.

### [(a) Polysaccharide Nanofiber]

The polysaccharide nanofibers (a) used are shown in Table 1.

**[Table 1]**

| (a) Polysaccharide nanofiber | | | | | |
|---|---|---|---|---|---|
| Polysaccharide nanofiber | Description | Viscosity /mPa·s | Specific surface area/m²/g | Degree of polymerization | Average fiber diameter/nm |
| CNF1: Cellulose nanofiber 1 | Powder | 6,000 | 120 | 650 | 10 to 50 |
| CNF2: Cellulose nanofiber 2 | | 700 | | 200 | |
| CNF3: Cellulose nanofiber 3 | 2% by weight Water dispersion | 7,000 | 120 | 800 | |
| CNF4: Cellulose nanofiber 4 | | 6,000 | | 650 | |
| CNF5: Cellulose nanofiber 5 | | 700 | 150 | 200 | |
| CnNF: Chitin nanofiber | | 3,000 | 200 | 300 | |
| CsNF: Chitosan nanofiber | | 2,000 | 80 | 480 | 20 to 50 |

The viscosity is a viscosity of an aqueous 2% by weight solution at 25°C.

### [(b) Acid Reactive Glass Powder]

FASG: Acid reactive glass powder
(fluoroaluminosilicate glass, 50% particle diameter: 3.2 µm)
[Production of FASG: Acid Reactive Glass Powder]

After mixing 23% by weight of silica, 8% by weight of aluminum oxide, 13% by weight of aluminum phosphate, 14% by weight of aluminum fluoride and 42% by weight of strontium carbonate in this proportion, the mixture was molten. The melt was quenched in water, and the glass thus obtained was ground to obtain an acid reactive glass powder. The 50% particle diameter of this acid reactive glass powder was measured with a laser diffraction type grain size measuring apparatus (Microtrac MT3300EXII: manufactured by NIKKISO Co., Ltd.), and the result was 3.4 µm.

### [(c) Polyalkenoic Acid]

PCA1: Acrylic acid-tricarboxylic acid copolymer powder 1
(weight average molecular weight: 250,000)
PCA2: Acrylic acid-tricarboxylic acid copolymer powder 2
(weight average molecular weight: 140,000)

### [(d) Water]

IEW: Ion-exchanged water
[Other Components]
TA: Tartaric acid

### [Preparation of Powder Material and Liquid Material]

Powder materials P1 to 8 were prepared by mixing respective components in the proportions shown in Table 2. Liquid materials L1 to 19 were prepared by mixing respective components in the proportions shown in Table 3. The dental glass ionomer cement compositions (Examples 1 to 28, Comparative Examples 1 to 6) prepared by kneading these powder materials and liquid materials in the combinations and at the powder/liquid ratios (by weight) shown in Examples and Comparative Examples were evaluated for compressive strength, working time and kneadability in accordance with the above-mentioned methods.

**[Table 2]**

| Powder material composition (% by weight) | | | | | | | | | |
|---|---|---|---|---|---|---|---|---|---|
| | | P1 | P2 | P3 | P4 | P5 | P6 | P7 | P8 |
| Component (a) | CNF1 | 0.050 | - | - | - | - | - | - | - |
| | CNF2 | - | 0.005 | 0.050 | 0.500 | 1.000 | 4.000 | 8.000 | - |
| Component (b) | FASG | 99.950 | 99.995 | 99.950 | 99.500 | 99.000 | 96.000 | 92.000 | 100.000 |
| Total | | 100.000 | 100.000 | 100.000 | 100.000 | 100.000 | 100.000 | 100.000 | 100.000 |

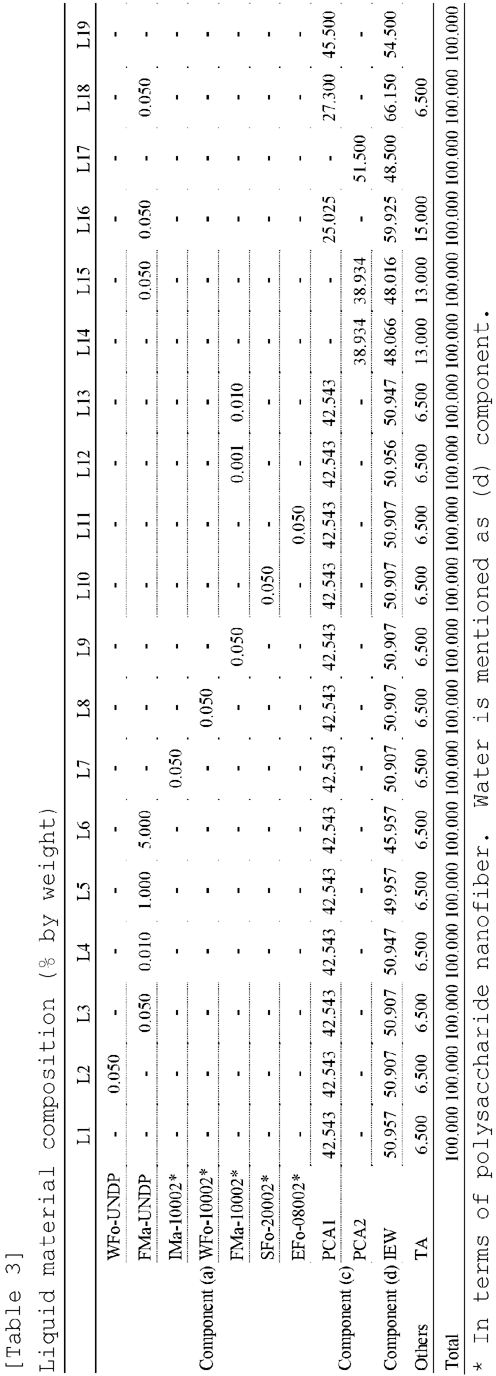

The evaluation results of the dental glass ionomer cement compositions of Examples 1 to 6 and Comparative Examples 1 to 6 are shown in Table 4.

In Examples 1 to 4, a very high compressive strength, sufficient working time, satisfactory kneadability and satisfactory dischargeability out of a container were exhibited, and therefore the compositions had desired properties as a dental glass ionomer cement.

In Examples 5 and 6, a high compressive strength, sufficient working time, satisfactory kneadability and satisfactory dischargeability out of a container were exhibited, and therefore the compositions had desired properties as a dental glass ionomer cement.

In Comparative Examples 1 to 3, although sufficient working time, satisfactory kneadability and satisfactory dischargeability out of a container were exhibited, the compressive strength was low.

In Comparative Examples 4 and 5, although a high compressive strength was exhibited, the working time was short. The viscosity of the kneaded material was high, and it was difficult to knead and discharge from a container.

In Comparative Example 6, although sufficient working time was exhibited, the compressive strength was low. The viscosity of the kneaded material was high, and it was difficult to knead and discharge from a container.

**[Table 4]**

| Combinations and evaluation results of Examples 1 to 6 and Comparative Examples 1 to 6 | | | | | | | | | | | | | |
|---|---|---|---|---|---|---|---|---|---|---|---|---|---|
| | | Example 1 | Example 2 | Example 3 | Example 4 | Example 5 | Example 6 | Comparative Example 1 | Comparative Example 2 | Comparative Example 3 | Comparative Example 4 | Comparative Example 5 | Comparative Example 6 |
| Powder material | | P1 | P2 | P3 | P4 | P5 | P6 | P7 | P8 | P8 | P8 | P8 | P7 |
| Liquid material | | L1 | L1 | L1 | L1 | L1 | L1 | L1 | L1 | L12 | L14 | L12 | L1 |
| Powder/liquid ratio | | 2.5/1.0 | 2.5/1.0 | 2.5/1.0 | 2.5/1.0 | 2.5/1.0 | 2.5/1.0 | 2.5/1.0 | 2.5/1.0 | 2.5/1.0 | 4.5/1.0 | 4.5/1.0 | 4.5/1.0 |
| Component (a) | WFo-UNDP | 0.0357 | - | - | - | - | - | - | - | - | - | - | - |
| | FMa-UNDP | - | 0.0036 | 0.0357 | 0.3571 | 0.7143 | 2.8571 | 5.7143 | - | - | - | - | 6.5455 |
| | Nanofiber in FMa-10002 | - | - | - | - | - | - | - | - | 0.0003 | - | 0.0002 | - |
| Component (b) | FASG | 71.3929 | 71.4250 | 71.3929 | 71.0715 | 70.7143 | 68.5715 | 65.7143 | 71.4286 | 71.4286 | 81.8182 | 81.8182 | 75.2727 |
| Component (c) | PCA1 | 12.1550 | 12.1550 | 12.1550 | 12.1550 | 12.1550 | 12.1550 | 12.1550 | 12.1550 | 12.1550 | - | 7.7350 | 7.7350 |
| | PCA2 | - | - | - | - | - | - | - | - | - | 7.0789 | - | - |
| Component (d) | IEW, and nanofiber-dispersed water | 14.5593 | 14.5593 | 14.5593 | 14.5593 | 14.5593 | 14.5593 | 14.5593 | 14.5593 | 14.5590 | 8.7393 | 9.2648 | 9.2650 |
| Others | TA | 1.8571 | 1.8571 | 1.8571 | 1.8571 | 1.8571 | 1.8571 | 1.8571 | 1.8571 | 1.8571 | 2.3636 | 1.1818 | 1.1818 |
| Total | | 100.0000 | 100.0000 | 100.0000 | 100.0000 | 100.0000 | 100.0000 | 100.0000 | 100.0000 | 100.0000 | 100.0000 | 100.0000 | 100.0000 |
| Compressive strength/MPa | | 238 | 258 | 258 | 245 | 219 | 210 | 185 | 192 | 189 | 205 | 201 | 193 |
| Working time/min:sec | | 3:20 | 3:30 | 3:30 | 3:20 | 3:30 | 3:40 | 4:00 | 3:30 | 3:30 | 1:10 | 1:00 | 2:50 |
| Kneadability | | A | A | A | A | A | A | A | A | A | C | C | C |
| Dischargeability out of container | | A | A | A | A | A | A | A | A | A | C | C | C |

The evaluation results of the dental glass ionomer cement compositions of Examples 7 to 17 are shown in Table 5.

In Examples 7 to 10, a very high compressive strength, sufficient working time, satisfactory kneadability and satisfactory dischargeability out of a container were exhibited, and therefore the compositions had desired properties as a dental glass ionomer cement.

In Example 11, a high compressive strength, sufficient working time, satisfactory kneadability and satisfactory dischargeability out of a container were exhibited, and therefore the composition had desired properties as a dental glass ionomer cement.

In Examples 12 to 17, a very high compressive strength, sufficient working time, satisfactory kneadability and satisfactory dischargeability out of a container were exhibited, and therefore the compositions had desired properties as a dental glass ionomer cement.

**[Table 5]**

| Combinations and evaluation results of Examples 7 to 17 | | | | | | | | | | | | |
|---|---|---|---|---|---|---|---|---|---|---|---|---|
| | | Example 7 | Example 8 | Example 9 | Example 10 | Example 11 | Example 12 | Example 13 | Example 14 | Example 15 | Example 16 | Example 17 |
| | Powder material | P8 | P8 | P8 | P8 | P8 | P8 | P8 | P8 | P8 | P8 | P8 |
| | Liquid material | L2 | L3 | L4 | L5 | L6 | L7 | L8 | L9 | L10 | L11 | L13 |
| | Powder/liquid ratio | 2.5/1.0 | 2.5/1.0 | 2.5/1.0 | 2.5/1.0 | 2.5/1.0 | 2.5/1.0 | 2.5/1.0 | 2.5/1.0 | 2.5/1.0 | 2.5/1.0 | 2.5/1.0 |
| Component (a) | WFo-UNDP | 0.0143 | - | - | - | - | - | - | - | - | - | - |
| | FMa-UNDP | - | 0.0143 | 0.0029 | 0.2857 | 1.4286 | - | - | - | - | - | - |
| | Nanofiber in IMa-10002 | - | - | - | - | - | 0.0143 | - | - | - | - | - |
| | Nanofiber in WFo-10002 | - | - | - | - | - | - | 0.0143 | - | - | - | - |
| | Nanofiber in FMa-10002 | - | - | - | - | - | - | - | 0.0143 | - | - | 0.0029 |
| | Nanofiber in SFo-20002 | - | - | - | - | - | - | - | - | 0.0143 | - | - |
| | Nanofiber in EFo-08002 | - | - | - | - | - | - | - | - | - | 0.0143 | - |
| Component (b) | FASG | 71.4286 | 71.4286 | 71.4286 | 71.4286 | 71.4286 | 71.4286 | 71.4286 | 71.4286 | 71.4286 | 71.4286 | 71.4286 |
| Component (c) | PCA1 | 12.1550 | 12.1550 | 12.1550 | 12.1550 | 12.1550 | 12.1550 | 12.1550 | 12.1550 | 12.1550 | 12.1550 | 12.1550 |
| Component (d) | IEW, and nanofiber-dispersed water | 14.5450 | 14.5450 | 14.5564 | 14.2736 | 13.1307 | 14.5450 | 14.5450 | 14.5450 | 14.5450 | 14.5450 | 14.5564 |
| Others | TA | 1.8571 | 1.8571 | 1.8571 | 1.8571 | 1.8571 | 1.8571 | 1.8571 | 1.8571 | 1.8571 | 1.8571 | 1.8571 |
| Total | | 100.0000 | 100.0000 | 100.0000 | 100.0000 | 100.0000 | 100.0000 | 100.0000 | 100.0000 | 100.0000 | 100.0000 | 100.0000 |
| Compressive strength/MPa | | 243 | 246 | 228 | 238 | 211 | 239 | 242 | 248 | 245 | 251 | 236 |
| Working time/min:sec | | 3:30 | 3:30 | 3:30 | 3:30 | 3:40 | 3:20 | 3:30 | 3:30 | 3:20 | 3:10 | 3:40 |
| Kneadability | | A | A | A | A | A | A | A | A | A | A | A |
| Dischargeability out of container | | A | A | A | A | A | A | A | A | A | A | A |

The evaluation results of the dental glass ionomer cement compositions of Examples 18 to 28 are shown in Table 6.

In Example 18, a high compressive strength, sufficient working time, satisfactory kneadability and satisfactory dischargeability out of a container were exhibited, and therefore the composition had desired properties as a dental glass ionomer cement.

In Examples 19 and 20, a very high compressive strength, sufficient working time, satisfactory kneadability and satisfactory dischargeability out of a container were exhibited, and therefore the compositions had desired properties as a dental glass ionomer cement.

In Example 21, a very high compressive strength and sufficient working time were exhibited. Although the viscosity of the kneaded material was slightly high, kneading and discharge from a container were possible without a problem, and therefore the composition had desired properties as a dental glass ionomer cement.

In Example 22, a high compressive strength and sufficient working time were exhibited. Although the viscosity of the kneaded material was slightly high, kneading and discharge from a container were possible without a problem, and therefore the composition had desired properties as a dental glass ionomer cement.

In Examples 23 and 24, a very high compressive strength, sufficient working time, satisfactory kneadability and satisfactory dischargeability out of a container were exhibited, and therefore the compositions had desired properties as a dental glass ionomer cement.

In Example 25, a high compressive strength, sufficient working time, satisfactory kneadability and satisfactory dischargeability out of a container were exhibited, and therefore the composition had desired properties as a dental glass ionomer cement.

In Example 26, a very high compressive strength, sufficient working time, satisfactory kneadability and satisfactory dischargeability out of a container were exhibited, and therefore the composition had desired properties as a dental glass ionomer cement.

In Example 27, a high compressive strength, sufficient working time, satisfactory kneadability and satisfactory dischargeability out of a container were exhibited, and therefore the composition had desired properties as a dental glass ionomer cement.

In Example 28, a very high compressive strength, sufficient working time, satisfactory kneadability and satisfactory dischargeability out of a container were exhibited, and therefore the composition had desired properties as a dental glass ionomer cement.

**[Table 6]**

| Combinations and evaluation results of Examples 18 to 28 | | | | | | | | | | | | |
|---|---|---|---|---|---|---|---|---|---|---|---|---|
| | | Example 18 | Example 19 | Example 20 | Example 21 | Example 22 | Example 23 | Example 24 | Example 25 | Example 26 | Example 27 | Example 28 |
| | Powder material | P3 | P3 | P8 | P8 | P8 | P8 | P3 | P3 | P8 | P8 | P3 |
| | Liquid material | L14 | L14 | L15 | L15 | L16 | L16 | L17 | L17 | L18 | L18 | L19 |
| | Powder/liquid ratio | 1.4/1.0 | 1.7/1.0 | 3.5/1.0 | 4.5/1.0 | 4.5/1.0 | 3.5/1.0 | 1.7/1.0 | 1.4/1.0 | 1.8/1.0 | 1.5/1.0 | 2.5/1.0 |
| Component (a) Component (b) | FMa-UNDP | 0.0292 | 0.0315 | 0.0111 | 0.0091 | 0.0091 | 0.0111 | 0.0315 | 0.0292 | 0.0179 | 0.0200 | 0.0357 |
| | FASG | 58.3041 | 62.9315 | 77.7778 | 81.8182 | 81.8181 | 77.7778 | 62.9314 | 58.3042 | 64.2857 | 60.0000 | 71.3929 |
| omponent (c) | PCA1 | - | - | - | - | 4.5500 | 5.5611 | - | - | 9.7500 | 10.9200 | 13.0000 |
| | PCA2 | 16.2225 | 14.4200 | 8.6520 | 7.0789 | - | - | 19.0741 | 21.4583 | - | - | - |
| Component (d) Others | IEW | 20.0275 | 17.8022 | 10.6702 | 8.7302 | 10.8955 | 13.3167 | 17.9630 | 20.2083 | 23.6250 | 26.4600 | 15.5714 |
| | TA | 5.4167 | 4.8148 | 2.8889 | 2.3636 | 2.7273 | 3.3333 | 0.0000 | 0.0000 | 2.3214 | 2.6000 | 0.0000 |
| Total | | 100.0000 | 100.0000 | 100.0000 | 100.0000 | 100.0000 | 100.0000 | 100.0000 | 100.0000 | 100.0000 | 100.0000 | 100.0000 |
| Compressive strength/MPa | | 207 | 223 | 247 | 243 | 212 | 221 | 227 | 210 | 222 | 209 | 252 |
| Working time/min: sec | | 4:00 | 3:50 | 2:10 | 1:30 | 2:20 | 2:40 | 2:00 | 1:30 | 3:50 | 4:10 | 2:50 |
| Kneadability | | A | A | A | B | B | A | A | A | A | A | A |
| Dischargeability out of container | | A | A | A | B | B | A | A | A | A | A | A |

## Claims

1. A dental glass ionomer cement composition comprising 0.001 to 3% by weight of (a) a polysaccharide nanofiber having a fiber aspect ratio of 100 or more and an average fiber diameter of 100 nm or less.

2. The dental glass ionomer cement composition according to claim 1, wherein the polysaccharide nanofiber (a) is at least one selected from the group consisting of a cellulose nanofiber, a chitin nanofiber and a chitosan nanofiber.

3. The dental glass ionomer cement composition according to claim 1 or 2, wherein the viscosity of an aqueous 2% by weight solution of the polysaccharide nanofiber (a) at 25°C is within a range of 500 to 10,000 mPa·s.

4. The dental glass ionomer cement composition according to any one of claims 1 to 3, comprising:
0.001 to 3% by weight of (a) a polysaccharide nanofiber,
60 to 80% by weight of (b) an acid reactive glass powder,
5 to 20% by weight of (c) a polyalkenoic acid, and
10 to 25% by weight of (d) water.
